(19) **Europäisches Patentamt / European Patent Office / Office européen des brevets**

(11) **EP 4 283 293 B1**

(12) **EUROPEAN PATENT SPECIFICATION**

(45) Date of publication and mention of the grant of the patent:
**27.05.2026   Bulletin 2026/22**

(21) Application number: **22926169.8**

(22) Date of filing: **26.08.2022**

(51) International Patent Classification (IPC):
**G01N 33/00** (2006.01)   **F23G 7/06** (2006.01)
**F23N 5/12** (2006.01)   **F23N 5/24** (2006.01)
**G06N 3/09** (2023.01)   **G05B 19/00** (2006.01)
**G05B 19/042** (2006.01)   **G05B 23/02** (2006.01)

(52) Cooperative Patent Classification (CPC):
**G01N 33/007; F23G 7/06; F23N 5/123; F23N 5/242;**
F23G 2209/14; G05B 19/0428; G05B 23/0297;
G06N 3/09

(86) International application number:
**PCT/KR2022/012790**

(87) International publication number:
**WO 2023/153571 (17.08.2023 Gazette 2023/33)**

(54) **VIRTUAL SENSOR SYSTEM FOR MEASURING VOC INFLOW AMOUNT OF RTO AND METHOD FOR VERIFYING VOC MEASUREMENT SENSOR BY USING SAME**

VIRTUELLES SENSORSYSTEM ZUR MESSUNG DER VOC-ZUFLUSSMENGE VON RTO UND VERFAHREN ZUR ÜBERPRÜFUNG EINES VOC-MESSSENSORS UNTER VERWENDUNG DAVON

SYSTÈME DE CAPTEUR VIRTUEL POUR LA MESURE D'UNE QUANTITÉ D'ENTRÉE DE COV D'UN RTO ET PROCÉDÉ DE VÉRIFICATION D'UN CAPTEUR DE MESURE DE COV À L'AIDE DE CE DERNIER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.02.2022   KR 20220018487**

(43) Date of publication of application:
**29.11.2023   Bulletin 2023/48**

(73) Proprietor: **LG Chem, Ltd.**
**Seoul 07336 (KR)**

(72) Inventors:
• **JEON, Ye Rin**
  **Daejeon 34122 (KR)**
• **CHOI, Jae Hoon**
  **Daejeon 34122 (KR)**
• **LEE, Chang Song**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
JP-A- 2007 138 921   KR-A- 20010 036 423
KR-A- 20210 019 909   KR-B1- 101 372 489
KR-B1- 101 894 541

**Description**

## TECHNICAL FIELD

The present invention relates to a virtual sensor system for measuring a VOC inflow amount of an RTO and a method for verifying a VOC measurement sensor using the same, more specifically, to a system and method for capable of verifying the reliability of the VOC measurement sensor using a two methods-based virtual sensor.

## BACKGROUND ART

A regenerative thermal oxidizer (hereinafter referred to as 'RTO') refers to a device that incinerates and removes volatile organic compounds (VOC) gas generated in a process. The RTO includes a combustion chamber and a predetermined number of beds made of a heat storage material to increase a heat recovery rate. On the other hand, since the RTO uses the thermal storage material, there is a risk of a safety accident occurring upon a sudden temperature change. In particular, when a high concentration of VOC is introduced, a rapid temperature increase may occur within a few seconds. Continuous monitoring of an amount of VOC flowing into the RTO is necessary. In order to solve this problem, in general, a method of monitoring the VOC inflow amount using a sensor that measures the amount of VOC flowing into the RTO in real time is being used. However, a flame ionization detector (FID) sensor, which is known to be the most reliable among VOC measuring sensors, may also cause a measurement error, which makes it difficult to accurately determine the VOC inflow amount.

As a related prior art, there is Korean Patent KR101607878B1 and the Korean patent application KR20210019909 A.

## DISCLOSURE OF THE INVENTION

## TECHNICAL PROBLEM

The present invention is to solve the problems described above, and to provide a virtual sensor system that can be used in parallel with the VOC measurement sensor to check a calibration time point and a method for diagnosing a VOC measurement sensor using the same.

## TECHNICAL SOLUTION

In order to solve the problems described above, the present invention provides a virtual sensor system for measuring an VOC inflow amount of an RTO in accordance with claim 1.

In addition, the present invention provides a method for verifying a FID VOC measurement sensor of an RTO using the virtual sensor system in accordance with claim 6.

## ADVANTAGEOUS EFFECTS

According to an embodiment of the present invention, a virtual sensor that can be used in parallel with the VOC measurement sensor to check the calibration time point and ultimately can replace the VOC measurement sensor is provided. Therefore, it is possible to reduce the risk of safety accidents by minimizing the measurement error of the existing VOC measurement sensor to accurately grasp the VOC inflow amount of the RTO.

## BRIEF DESCRIPTION OF THE DRAWINGS

FIG. 1 is a diagram schematically illustrating the overall configuration of a virtual sensor system for measuring a VOC inflow amount of an RTO according to an embodiment of the present invention.
FIG. 2 is a diagram schematically illustrating an internal configuration of the RTO and an RTO control device according to an embodiment of the present invention.
FIG. 3 is a diagram illustrating a first VOC calculation model training process according to an embodiment of the present invention.
FIG. 4 is a diagram illustrating an example of a result of applying the first VOC calculation model according to an embodiment of the present invention.
FIGS. 5 and 6 are diagrams illustrating examples of VOC sensor reliability verification criteria according to an embodiment of the present invention.

## MODE FOR CARRYING OUT THE INVENTION

Hereinafter, embodiments of the present invention will be described in detail with reference to the accompanying drawings so that those of ordinary skill in the art can easily embody the present invention. However, the present invention may be implemented in various different forms and is not limited to the embodiments described herein. In order to clearly describe the present invention in the drawings, parts irrelevant to the description are omitted, and similar reference numerals are attached to similar parts throughout the specification.

Hereinafter, the present invention will be described in detail with reference to the drawings.

1. Terms used in the present invention.

1.1. RTO operation data/derived factor

The RTO operation data refers to measurement values of various sensors disposed inside the RTO during RTO operation.

Specifically, the RTO operation data may include an RTO blower driving power amount, an LNG fuel input amount, a bed inlet temperature/pressure, a chamber temperature, a chamber outlet temperature, bed upper/lower temperature, etc. Accordingly, various sensors may be composed of sensors necessary for measuring operation data, such as a blower power amount sensor, a LNG fuel amount sensor, a bed inlet temperature/pressure sensor, a chamber temperature sensor, a chamber outlet temperature sensor, and a bed upper/lower temperature sensor.

Meanwhile, the derived factors are values acquired from the RTO operation data, and may include a chamber temperature change (dT), a bed upper/lower temperature difference (Tbed_diff), etc.

1.2. VOC sensor

The VOC sensor refers to a physical sensor that is disposed in each of a plurality of RTOs and measures the amount of VOC flowing into the corresponding RTO in real time. In the present invention, the VOC sensor may be implemented as a commonly known flame ionization detector (FID).

In the present invention, the 'VOC sensor' may be used interchangeably with a 'FID sensor unit'.

1.3. virtual sensor

The virtual sensor is used in parallel with the VOC sensor to check a calibration time point, and is a sensor developed to ultimately replace the physical sensor. The virtual sensor is implemented based on the concept of inversely calculating a VOC inflow amount using the RTO operation data.

This virtual sensor is composed of a training-based virtual sensor that estimates the VOC inflow amount using machine learning techniques using the RTO operation data and derived factors obtained therefrom and a theory-based virtual sensor that is based on the First Principle and predicts VOC inflow amount/concentration based on a difference between fuel combustion energy and hot air temperature rise energy generated inside the RTO.

2. Virtual sensor system for measuring VOC inflow amount of RTO according to the present invention

The virtual sensor system for measuring the VOC inflow amount of the RTO according to the present invention is a system for monitoring the VOC inflow amount of a each regenerative thermal oxidizer (RTO) of a plurality of regenerative thermal oxidizers RTO1, RTO2,..., RTOn.

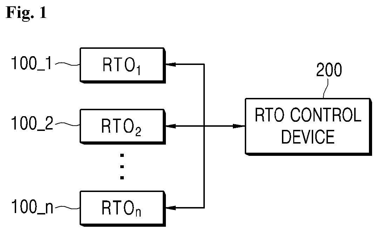

FIG. 1 is a diagram schematically illustrating the overall configuration of the virtual sensor system for measuring the VOC inflow amount of the RTO according to an embodiment of the present invention.

Referring to FIG. 1, the system of the present invention is largely configured with a plurality of RTOn 100_1, 100_2,..., 100_n and a RTO control device 200 for controlling the RTOns.

2.1. Regenerative thermal oxidizer (RTO)

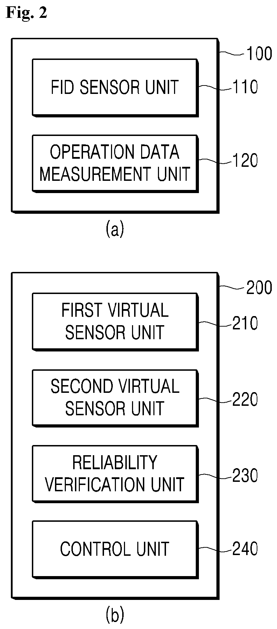

FIG. 2(a) is a diagram schematically illustrating the internal configuration of the RTO according to an embodiment of the present invention.

Referring to FIG. 2(a), respective RTOn 100_1, 100_2,.., 100_n included in the system of the present invention may include the following configuration.

2.1.1. FID sensor unit 110

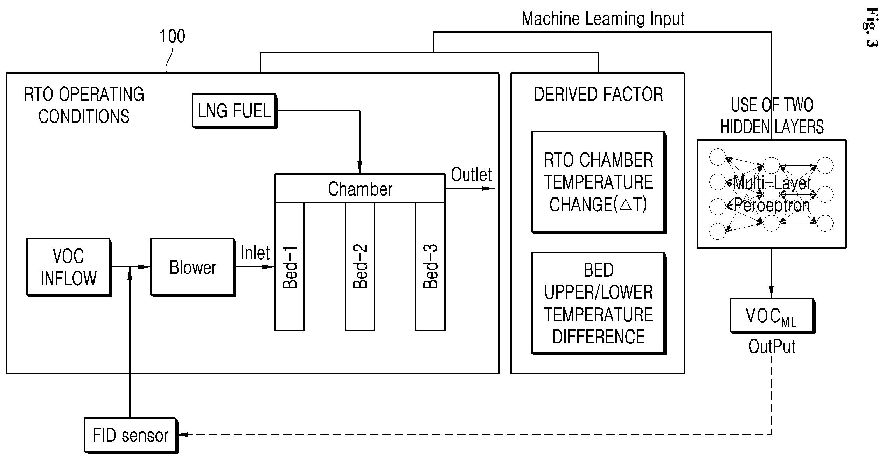

The FID sensor unit 110 is composed of the flame ionization detector (FID) and is a VOC sensor that periodically measures the VOC inflow amount in real time. For example, as illustrated in FIG. 3, it may be disposed near a blower to detect/measure the amount of VOC introduced. A value measured by the FID sensor unit 110 may be referred to as an FID sensor measurement value, a VOC sensor measurement value, or VOC1 hereinafter.

2.1.2. Operation data measurement unit 120

The operation data measurement unit 120 is configured with various sensors necessary for measuring RTO operation data including the RTO blower driving power amount, the LNG fuel input amount, the bed inlet temperature/-pressure, the chamber temperature, the chamber outlet temperature, the bed upper/lower temperature, etc., and is configured to measure the operation data in real time at regular intervals.

2.2. RTO control device 200

FIG. 2(b) is a diagram schematically illustrating the internal configuration of the RTO control device according to an embodiment of the present invention.
Referring to FIG. 2(b), the RTO control device 200 of the present invention may include the following configuration.

2.2.1. First virtual sensor unit 210

The first virtual sensor unit 210 is a training-based virtual sensor for estimating the VOC inflow amount by machine learning by utilizing the RTO operation data.
More specifically, the first virtual sensor unit 210 generates a first VOC calculation model using the RTO operation data and FID sensor measurement values acquired from each RTO, and estimates/predicts the VOC inflow amount from operation data of each RTO through the generated first VOC calculation model as an output value of the training-based sensor.
The first virtual sensor unit 210 may be configured to include the following detailed configuration.

a. first operation data input unit

The first operation data input unit is configured to receive operation data measured in real time from the operation data measurement unit 120 of each of the plurality of RTOns 100_1, 100_2,..., 100_n.

b. VOC inflow amount estimation ML training unit

The VOC inflow amount estimation ML training unit generates the first VOC calculation model, which is a VOC inflow amount estimation model, by performing machine learning in which operation data of each RTO received from the first operation data input unit and the derived factor generated from the operation data are used as an input and the FID sensor measurement value of the FID sensor unit 110 is used as an output.
FIG. 3 is a diagram illustrating a first VOC calculation model training process according to an embodiment of the present invention.
Referring to FIG. 3, a process of training the first VOC calculation model is performed in a way of inputting the input variable composed of RTO operation data and the derived factor generated therefrom to two hidden layers and conducting supervised learning with the FID sensor measurement value VOC1 as a true value.
That is, the first VOC calculation model is an artificial intelligence neural network model trained by using the VOC inflow measurement values of the FID sensor unit 110 and the RTO operation data corresponding to the VOC inflow measurement values.
Here, the derived factor may include the chamber temperature change dT acquired from the chamber temperature of the RTO operation data, and the bed upper and lower temperature difference acquired from the bed upper/lower temperatures, as described above.
Such a first virtual sensor unit 210 estimates/predicts the VOC inflow amount of the corresponding RTO from the RTO operation data acquired from the plurality of RTOns 100_1, 100_2,..., 100_n using the trained first VOC calculation model, and the output value thereof may be referred to as a training-based virtual sensor prediction value VOC2.

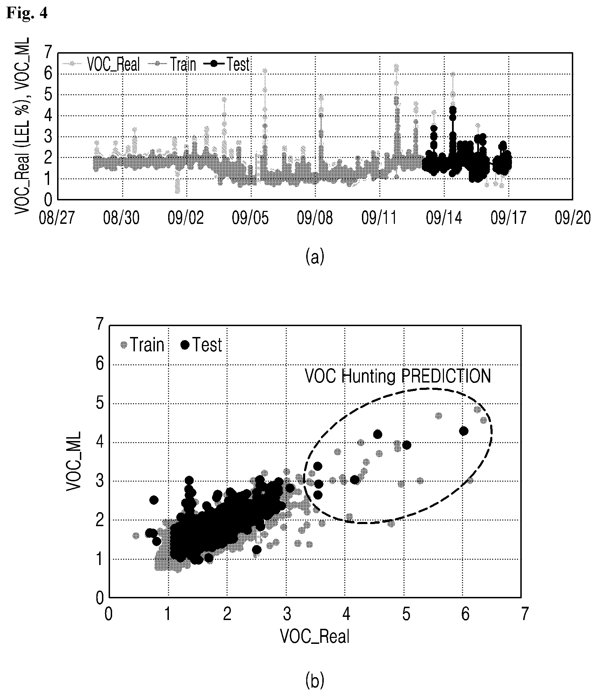

FIG. 4 is a diagram illustrating an example of a result of applying the trained VOC inflow amount estimation model (first VOC calculation model) according to an embodiment of the present invention.

When looking at the graph shown in FIG. 4 (a), the light gray graph is the VOC sensor measurement value over a period of time, the dark grey graph is the VOC inflow amount predicted value in the supervised learning process using the VOC sensor measurement values and RTO operation data corresponding to the VOC sensor measurement values, and the black graph shows the VOC inflow amount estimated/calculated from actual RTO operation data by using the trained model. When the actual measured value (light gray graph) of the VOC sensor, which is a physical sensor, and the predicted value (black graph) from the trained model are mutually compared with each other, it is possible to check a time point at which a difference of a predetermined reference value or more occurs, and through this, the VOC sensor measurement error can be minimized by diagnosing/verifying the VOC sensor measurement value at the corresponding time point. In addition, as illustrated in FIG. 4(b), it is possible to check the calibration time point through the comparison between the actual measured value of the VOC sensor and the VOC inflow predicted value estimated by applying the trained model, and thus the diagnosis/verification of the VOC sensor measurement value can be performed to reduce the measurement error.

2.2.2. Second virtual sensor unit 220

The second virtual sensor unit 220 is a theory-based virtual sensor equipped with a second VOC calculation model for calculating the VOC inflow amount of RTO by using VOC combustion energy. More specifically, it is configured to predict the VOC inflow amount and/or concentration based on the difference between the combustion energy of fuel and the hot air temperature rise energy generated inside the RTO.

The second virtual sensor unit 220 may be configured to include the following detailed configuration.

a. Second operation data input unit

The second operation data input unit is configured to receive RTO operation data measured in real time from the operation data measurement unit 120 of each of the plurality of RTOns 100_1, 100_2,..., 100_n.

b. Combustion energy calculation unit

The combustion energy calculation unit calculates combustion energy $Q_1$ of the fuel LNG by the following.

$$\text{Equation 1}$$

$$Q1 = LNG\ input \times 43{,}950$$

Here, 43,950 refers to the specific heat capacity, which is the heat energy required to raise the temperature of a unit mass of matter by 1 degree. The energy generated by combustion per $1Nm^3$ of LNG fuel is 43.95 MJ. This was converted into a unit kJ and calculated and a value of 43,950 is used use as the specific heat capacity. Each unit is LNG input amount $[Nm^3/hr]$ and 43,950 $[kJ/Nm^3]$, and thus the unit of combustion energy of LNG fuel is $[kJ/hr]$.

On the other hand, the LNG input amount (flow rate) can be measured by using a conventional LNG inflow amount measuring instrument (sensor). Although not illustrated in the drawings, the LNG inflow amount measurement sensor may be disposed in a pipe directly connected to the RTO to measure the flow rate of LNG. For complete combustion, since it is necessary to adjust the amount of air input along with the amount of LNG input, the flow sensor is typically preferably located near the RTO.

c. Hot air temperature rise energy before/after combustion calculation unit

The hot air temperature rise energy before/after combustion calculation unit calculates energy Q2 required for the temperature rise of the air flowing into the RTO by Equation 2 below.

$$\text{Equation 2}$$

$$Q2 = m \times Cp \times (T1 - T2)$$

Here, m means the air flow rate. This is calculated from differential pressure data of the air flow sensor or a pressure sensor at an input end and a pressure sensor at an output end of the blower.

Further, Cp is computed from $\dfrac{101{,}325}{286.9\ X\ (air\ temperature + 273.15)}$. This is the part that calculates the density of air using the ideal gas equation (pressure = density × gas constant × temperature). 101,325 (Pa) is the pressure, 286.9 (kJ/kg) is the gas constant, and 273.15 is the absolute zero value. However, the above Equation 2 corresponds to the case where the unit of the air flow rate m is [kg/hr]. When the unit of the air flow rate m is [m$^3$/hr], the above Equation 2 may be further multiplied by an air density value (e.g., 120).

Further, T1 is the temperature of the bed inlet temperature, and T2 is the the temperature after combustion, which is the chamber outlet temperature. Since a structure in which the air introduced into the blower flows into the bed inlet and exits to the chamber outlet in the RTO chamber is adopted, temperature sensors are disposed at respective locations of the bed inlet and chamber outlet to measure the temperatures before/after combustion, respectively. More specifically, when the heat storage material is configured in two stages, the temperature sensor located inside the RTO is usually located before/after passing the first heat storage material, after passing the second heat storage material, in a combustion chamber, etc. Thus, according to an embodiment of the present invention, the combustion chamber temperature, the temperature after combustion, the temperature after passing the second heat storage material, the temperature before combustion, that is, the temperature value immediately after passing through all the heat storage materials may be used.

d. VOC inflow amount calculation unit

The VOC inflow amount calculation unit calculates the VOC inflow amount by Equation 3 below by using the combustion energy Q1 calculated by the combustion energy calculation unit and the energy Q2 required for the temperature rise of the air flowing into the RTO calculated in the hot air temperature rise energy before/after combustion calculation unit.

Equation 3

$$VOC\ inflow\ amount\ = \frac{Q3}{VOC\ heat\ of\ combustion}$$

Here, Q3 = Q2 - Q1, which refers to the value acquired by subtracting the combustion energy from hot air temperature rise before/after combustion.
On the other hand, the VOC heat of combustion is different for each VOC material, and can be obtained in a known manner using standard enthalpy of formation.

e. LEL concentration calculation unit

The second virtual sensor unit may further include an LEL concentration calculation unit for calculating the LEL concentration (%) of the VOC by Equation 4 below.

Equation 4

$$LEL(\%) = \frac{(Q2 - Q1)X\ 0.016}{air\ flow\ rate\ X\ 1.93\ X\ \frac{101,325}{286.9\ X\ (air\ temperature\ + 273.15)}}$$

$$= \frac{(VOC\ inflow\ amount)}{(hot\ air\ inflow\ amount)}$$

Here, (Q2 - Q1) is the difference between the hot air temperature rise energy before/after combustion Q2 calculated by Equation 2 and the combustion energy Q1 calculated by Equation 1 above, that is, energy generated by the combustion of VOC.
Further, 1.93 is a value indicating the density of the VOC substance Butene. Since the density of Butene is about 1.93 times that of air, the density of VOC (Butene) is obtained by multiplying the part $\frac{101,325}{286.9\ X\ (air\ temperature\ + 273.15)}$,

which is a part to obtain the density of air, by 1.93.
Further, in the case of Butene, LEL 100% is 1.6%. LEL 1% means that the ratio of air and Butene is 0.016%, that is, it means that 0.00016 m$^3$/hr of Butene is contained when the air flow rate is 100 m$^3$/hr.
Here, 1.93 and 0.016 correspond to the case where the VOC material is Butene, and they vary depending on the VOC material.

In the case of the LEL concentration (%) of VOC, since it can be calculated through the ratio of the VOC inflow amount and the air flow rate calculated through Equations 1 to 3 above, it can be set to obtain the VOC inflow amount or LEL concentration (%) as an output value of the theory-based virtual sensor, which is the final output value of the second virtual sensor unit 220, if necessary.

### 2.2.3. Reliability verification unit 230

The reliability verification unit 230 has a configuration in which the VOC sensor measurement value is received from each of the plurality of RTOns 100_1, 100_2,..., 100_n, and based on this, the VOC sensor reliability of the RTO or the presence or absence of the VOC sensor (FID sensor unit 110) is verified.

As a reference for verifying the VOC sensor reliability, if the VOC sensor measurement value satisfies any one of the following two cases, it may be determined that the corresponding VOC sensor reliability is low.

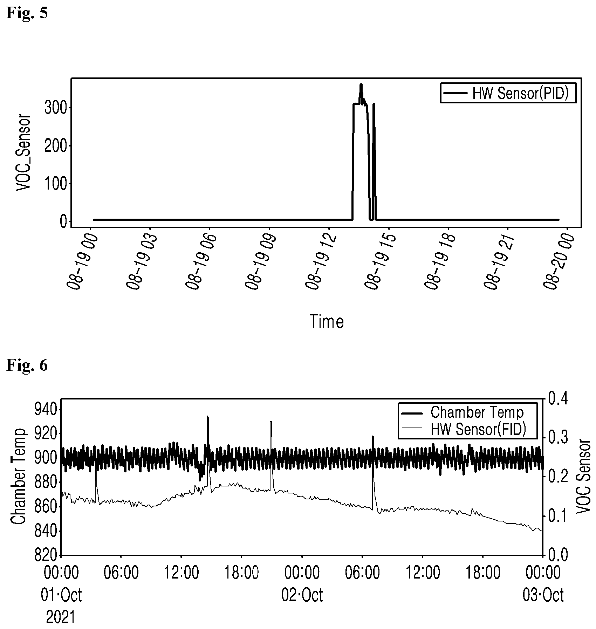

FIGS. 5 and 6 are diagrams illustrating an example of determining that the VOC sensor reliability is low according to an embodiment of the present invention.

As a first case, as illustrated in FIG. 5, when the VOC sensor measurement value does not change for a predetermined time or more, it may be determined that the reliability of the VOC sensor is low.

As a second case, as illustrated in FIG. 6, if a case where the VOC sensor measurement value exceeds the reference value occurs by a predetermined number of times or more even though there is no change beyond the reference range in the RTO operation data, it may be determined that the reliability of the corresponding VOC sensor is low. Here, in the case of FIG. 6, the change in the chamber temperature value among the RTO operation data is displayed in thick line, and the VOC sensor measurement value is displayed in a thin line.

When any one of the first and second cases is satisfied, it is determined that the VOC sensor reliability of the corresponding RTO is low, and, for example, a low signal indicating the same may be output. On the other hand, when both the first and second cases are not applicable, it is determined that the VOC sensor reliability of the corresponding RTO is high, and, for example, a high signal indicating the same may be output.

### 2.2.4. Control unit 240

The control unit 240 is configured to acquire the calculated value of VOC inflow amount from any one of the first virtual sensor unit 210 and the second virtual sensor unit 220 according to the verification result of the reliability verification unit 230 and the presence or absence of the FID sensor for each of RTOs 100_1, 100_2,..., 100_n, and generate a diagnostic alarm for the VOC sensor measurement value of the corresponding RTO through mutual comparison between the acquired VOC inflow amount from the virtual sensor with the VOC sensor measurement value of the FID sensor unit 110.

### a. Virtual sensor selection unit

The virtual sensor selection unit may select any one of the first virtual sensor unit 210 and the second virtual sensor unit 220 according to the verification result of the reliability verification unit 230 and the presence or absence of the FID sensor for each of RTOs 100_1, 100_2,..., 100_n, and calculate and acquire the VOC inflow amount therefrom.

Specifically, when an FID sensor exists and it is determined that the reliability of the VOC sensor is high (when a high signal is output) as a result of the verification of the reliability verification unit 230, the virtual sensor prediction value of the corresponding RTO may be calculated/acquired from the VOC inflow amount from the first virtual sensor unit 210.

On the other hand, when the FID sensor does not exist or it is determined that the reliability of the VOC sensor is low (when a low signal is output) as a result of the verification of the reliability verification unit 230, the virtual sensor prediction value of the corresponding RTO may be calculated/acquired from the VOC inflow amount from the second virtual sensor unit 220.

### b. Diagnostic alarm generation unit

The diagnostic alarm generation unit mutually compares the VOC inflow amount from the first virtual sensor unit or the second virtual sensor unit acquired by the virtual sensor selection unit with the VOC inflow measurement value of the FID sensor of the corresponding RTO at the same time point, and generates a diagnostic alarm for the VOC sensor measurement value according to the comparison result.

As a result of the comparison, when there is a difference between the VOC inflow amount from the first virtual sensor unit 210 or the second virtual sensor unit 220 and the VOC sensor measurement value by a predetermined reference value or more, a diagnostic alarm may be generated to prompt the user to verify the corresponding VOC sensor measurement value.

3. Method for verifying VOC measurement sensor of RTO using virtual sensor according to the present invention

The system for verifying the VOC measurement sensor of the RTO using the virtual sensor according to an embodiment of the present invention, as described above, is configured to include the plurality of RTOns 100_1, 100_2,..., 100_n and the RTO control device 200 for controlling the RTOns. The method for verifying the VOC measurement sensor of such a system may include the following steps

3.1. Data measurement and collection step

The data measurement and collection step is a step of collecting operation data and VOC sensor measurement values measured in real time at regular intervals from each of the plurality of RTOns 100_1, 100_2,..., 100_n.
More specifically, real-time VOC sensor measurement values and RTO operation data are collected from the FID sensor unit 110 and the operation data measurement unit 120, which is composed of various sensors, provided for each of the RTOs 100_1, 100_2,..., 100_n.

3.2. VOC sensor verification and virtual sensor selection step

The VOC sensor verification and virtual sensor selection step is a step of verifying the reliability of the VOC sensor of the corresponding RTO based on the collected VOC sensor measurement values for each of the RTOs 100_1, 100_2,..., 100_n and selecting one of the training-based virtual sensor and the theory-based virtual sensor based on the verification result.
Specifically, when any one of a case where the VOC sensor measurement value does not change for a predetermined time or more and a case where the VOC sensor measurement value exceeds the reference value occurs by a predetermined number of times or more even though there is no change beyond the reference range in the RTO operation data is satisfied, it may be determined that the reliability of the VOC sensor of the corresponding RTO is low. Since such a VOC sensor reliability verification reference has been described in detail with reference to FIGS. 5 and 6 in the system configuration above, a detailed description thereof will be omitted.
Meanwhile, when it is determined that the reliability of the VOC sensor measurement value is high as a result of the verification, the training-based virtual sensor equipped with the first VOC calculation model trained to estimate the VOC inflow amount using the RTO operation data is selected. On the other hand, when it is determined that the reliability of the VOC sensor measurement value is low, the theory-based virtual sensor equipped with the second VOC calculation model that calculates the VOC inflow amount using VOC combustion energy is selected. Here, it is set to select the theory-based virtual sensor even when the VOC sensor does not exist.
For example, when it is determined that the sensor reliability of the second RTO 100_2 is high, the training-based virtual sensor is selected, and when it is determined that the sensor reliability is low, the theory-based virtual sensor is selected.
The virtual sensor selected in this way can be used to determine whether or not diagnosis of the VOC sensor value of the RTO is necessary by using the predicted value obtained from the corresponding virtual sensor in a step to be described later.

3.3. Virtual sensor prediction value acquisition step

The virtual sensor prediction value acquisition step is a step of acquiring the predicted value from the selected virtual sensor for each RTO in the VOC verification and virtual sensor selection step.
When the selected virtual sensor is the training-based virtual sensor, the VOC inflow amount estimated using the operation data of the RTO through the first VOC calculation model from training-based virtual sensor can be acquired as a virtual sensor prediction value VOC2.
Here, the first VOC calculation model is an artificial intelligence neural network model generated by inputting input variables composed of RTO operation data and derived factors generated therefrom into two hidden layers, and conducting supervised learning by using the VOC sensor measurement value VOC1 as a true value.
On the other hand, when the selected virtual sensor is the theory-based virtual sensor, the VOC inflow amount calculated from the VOC combustion energy of the RTO through the second VOC calculation model using Equations 1 to 4 above can be acquired as a virtual sensor prediction value VOC3.
Through these steps, the VOC inflow amount can be acquired from the selected virtual sensor according to the VOC sensor reliability level for each RTO.

3.4. Sensor data comparison step

The sensor data comparison step is a step of mutually comparing the VOC inflow amount, which is the virtual sensor prediction value acquired for each RTO through the virtual sensor prediction value acquisition step, with the collected VOC sensor measurement values at the same time point.

For example, a VOC sensor measurement value VOC1_2 and a training-based virtual sensor prediction value VOC2_2 or a theory-based virtual sensor prediction value VOC3_2 at time t of the second RTO 100_2 are mutually compared with each other.

3.5. Diagnostic alarm generation step

The diagnostic alarm generation step is a step of generating a diagnostic alarm for the VOC sensor value of the corresponding RTO when there is a difference between the virtual sensor prediction value and the VOC sensor measurement value by a predetermined reference value or more, as a result of the comparison.

When there is a difference between the VOC sensor measurement value and virtual sensor prediction value of the RTO by a predetermined reference value or more, a diagnostic alarm is generated to prompt the user to verify the VOC sensor measurement value.

Meanwhile, although the technical idea of the present invention has been described in detail according to the above embodiment, it should be noted that the above embodiment is for the purpose of description and not for limitation. Further, those skilled in the art will understand that various embodiments are possible within the scope of the present invention.

**Claims**

1. A virtual sensor system for measuring a volatile organic compounds (VOC) inflow amount of a regenerative thermal oxidizer (RTO), comprising:

   a plurality of RTOs (Regenerative Thermal Oxidizer) (100_n) and
   an RTO control device (200) for controlling the RTOs (100_n),
   **characterized in that**
   each RTO (100_n) comprises an operation data measurement unit (120) configured to measure RTO operation data in real time at regular intervals, and a flame ionization detector (FID (110), 110) for measuring periodically the VOC inflow amount in real time,
   wherein the RTO control device (200) comprises

   a reliability verification unit (230) that verifies the presence or absence or reliability of the FID (110),
   a first virtual sensor unit (210) equipped with a first VOC calculation model for estimating the VOC inflow amount from RTO operation data, the first VOC calculation model being a machine learning model trained with the operation data of the RTO (100_n) and the amount of VOC measured by the FID (110) as true value,
   a second virtual sensor unit (220) equipped with a second VOC calculation model for predicting the VOC inflow amount based on the difference between the combustion energy of the fuel and the air temperature rise in the RTO(100_n),
   a control unit (240) that may select any one of the first virtual sensor unit (210) and the second virtual sensor unit (220) according to the verification result of the reliability verification unit (230) and the presence or absence of the FID (110) for each RTO(100_n) and calculate and acquire the VOC inflow amount therefrom.

2. The virtual sensor system of claim 1, wherein
   the reliability verification unit (230) determines that
   the reliability of the FID (110) is low if any one of the following conditions is met: a case where a VOC inflow measurement value input from the FID (110) of each RTO(100_n) does not change for a predetermined time or a case in which the VOC inflow measurement value exceeds a reference value even though there is no change beyond the reference range in the RTO operation data.

3. The virtual sensor system of claim 1, wherein
   the control unit (240) selects the first virtual sensor unit (210) when it is determined that the FID (110) exists and the reliability of the FID (110) is high, and selects the second virtual sensor unit (220) when it is determined that the FID (110) does not exist or the reliability of the FID (110) is low.

4. The virtual sensor system of claim 1, wherein
the control unit (240) further comprises
a diagnostic alarm generation unit that compares the VOC inflow amount acquired from the selected virtual sensor unit with the VOC inflow measurement value of the FID (110) at the same time point, and generates a diagnostic alarm for the VOC inflow measurement value based on the comparison result.

5. The virtual sensor system of claim 1, wherein
the first VOC calculation model of the first virtual sensor unit (210) is an artificial intelligence-based neural network model.

6. A method for verifying an FID (110) of an RTO (100_n) using a virtual sensor system according to claim 1, the method comprises:

a data measurement and collection step, which includes collecting operation data and a measurement value from the FID (110) measured in real time at regular intervals from each of a plurality of RTOs (100_n);
an FID (110) verification step and virtual sensor selection step, which includes verifying reliability of the FID (110) of the RTO (100_n) based on the collected measurement values of the FID (110) for each RTO(100_n) and selecting one of the first virtual sensor unit (210) and the second virtual sensor unit (220) based on the verification result;
a virtual sensor prediction value acquisition step, which includes acquiring a calculated value of VOC inflow amount from the virtual sensor selected for each RTO (100_n) in the FID (110) verification and virtual sensor selection step;
a sensor data comparison step, which includes mutually comparing the calculated value of VOC inflow amount acquired for each RTO (100_n) through the virtual sensor prediction value acquisition step and the collected measurement value from the FID (110) at the same time point; and
a diagnostic alarm generation step, which includes generating a diagnostic alarm for the measurement value from the FID (110) when there is a difference between the calculated value of VOC inflow amount and the measurement value from the FID (110) by a predetermined reference value or more as a result of the comparison.

7. The method of claim 6, wherein in the FID (110) verification and virtual sensor selection step, the first virtual sensor unit (210) is selected if the FID (110) exists and the reliability of the FID (110) is greater than or equal to a predetermined reference and the second virtual sensor unit (220) is selected if the FID (110) does not exist or the reliability of the FID (110) is less than the predetermined reference value.

8. The method of claim 7, wherein the first virtual sensor unit (210) is configured to calculate the VOC inflow amount using RTO operation data through an artificial intelligence neural network model trained using the measurement values from the FID (110) and the RTO operation data corresponding to the measurement values from the FID (110).

9. The method of claim 7, wherein the second virtual sensor unit (220) predicts the VOC inflow amount based on the difference between the combustion energy of the fuel and the air temperature rise inside the RTO (100_n).

**Patentansprüche**

1. Virtuelles Sensorsystem zum Messen einer Zuflussmenge flüchtiger organischer Verbindungen (VOC) eines regenerativen thermischen Oxidationsmittels (RTO), umfassend:

mehrere RTOs (regeneratives thermisches Oxidationsmittel) (100_n) und
eine RTO-Steuervorrichtung (200) zum Steuern der RTOs (100_n),
**dadurch gekennzeichnet, dass**
jedes RTO (100_n) eine Betriebsdatenmesseinheit (120), die konfiguriert ist, um RTO-Betriebsdaten in Echtzeit in regelmäßigen Intervallen zu messen, und einen Flammenionisationsdetektor (FID (110), 110) zum periodischen Messen der VOC-Zuflussmenge in Echtzeit umfasst,
wobei die RTO-Steuervorrichtung (200) umfasst

eine Zuverlässigkeitsverifizierungseinheit (230), die das Vorhandensein oder Nichtvorhandensein oder die Zuverlässigkeit des FID (110) verifiziert,
eine erste virtuelle Sensoreinheit (210), die mit einem ersten VOC-Berechnungsmodell zum Schätzen der VOC-Zuflussmenge aus RTO-Betriebsdaten ausgestattet ist, wobei das erste VOC-Berechnungsmodell ein Maschinenlernmodell ist, das mit den Betriebsdaten des RTO (100_n) und der Menge an VOC, die durch den FID (110) als wahrer Wert gemessen wird, trainiert ist,

eine zweite virtuelle Sensoreinheit (220), die mit einem zweiten VOC-Berechnungsmodell zum Vorhersagen der VOC-Zuflussmenge basierend auf der Differenz zwischen der Verbrennungsenergie des Kraftstoffs und dem Lufttemperaturanstieg in dem RTO (100_n) ausgestattet ist,

eine Steuereinheit (240), die eine beliebige der ersten virtuellen Sensoreinheit (210) und der zweiten virtuellen Sensoreinheit (220) gemäß dem Verifizierungsergebnis der Zuverlässigkeitsverifizierungseinheit (230) und dem Vorhandensein oder Nichtvorhandensein des FID (110) für jedes RTO (100_n) auswählen und die VOC-Zuflussmenge daraus berechnen und erfassen kann.

2. Virtuelles Sensorsystem nach Anspruch 1, bei dem
die Zuverlässigkeitsverifizierungseinheit (230) bestimmt, dass
die Zuverlässigkeit des FID (110) niedrig ist, wenn eine der folgenden Bedingungen erfüllt ist: ein Fall, in dem sich ein VOC-Zuflussmesswert, der von dem FID (110) jedes RTO (100_n) eingegeben wird, für eine vorbestimmte Zeit nicht ändert, oder ein Fall, in dem der VOC-Zuflussmesswert einen Referenzwert überschreitet, obwohl es keine Änderung über den Referenzbereich hinaus in den RTO-Betriebsdaten gibt.

3. Virtuelles Sensorsystem nach Anspruch 1, bei dem
die Steuereinheit (240) die erste virtuelle Sensoreinheit (210) auswählt, wenn bestimmt wird, dass der FID (110) vorhanden ist und die Zuverlässigkeit des FID (110) hoch ist, und die zweite virtuelle Sensoreinheit (220) auswählt, wenn bestimmt wird, dass der FID (110) nicht vorhanden ist oder die Zuverlässigkeit des FID (110) niedrig ist.

4. Virtuelles Sensorsystem nach Anspruch 1, bei dem
die Steuereinheit (240) ferner umfasst
eine Diagnosealarmerzeugungseinheit, die die VOC-Zuflussmenge, die von der ausgewählten virtuellen Sensoreinheit erfasst wird, mit dem VOC-Zuflussmesswert des FID (110) zum gleichen Zeitpunkt vergleicht und einen Diagnosealarm für den VOC-Zuflussmesswert basierend auf dem Vergleichsergebnis erzeugt.

5. Virtuelles Sensorsystem nach Anspruch 1, bei dem
das erste VOC-Berechnungsmodell der ersten virtuellen Sensoreinheit (210) ein auf künstlicher Intelligenz basierendes neuronales Netzwerkmodell ist.

6. Verfahren zum Verifizieren eines FID (110) eines RTO (100_n) unter Verwendung eines virtuellen Sensorsystems nach Anspruch 1, wobei das Verfahren umfasst:

einen Datenmess- und -sammelschritt, der das Sammeln von Betriebsdaten und eines Messwerts von dem FID (110), die in Echtzeit in regelmäßigen Intervallen gemessen werden, von jedem von mehreren RTOs (100_n) umfasst;

einen FID(110)-Verifizierungsschritt und einen Virtuelle-Sensor-Auswahlschritt, der das Verifizieren der Zuverlässigkeit des FID (110) des RTO (100_n) basierend auf den gesammelten Messwerten des FID (110) für jedes RTO (100_n) und das Auswählen einer der ersten virtuellen Sensoreinheit (210) und der zweiten virtuellen Sensoreinheit (220) basierend auf dem Verifizierungsergebnis umfasst;

einen Virtuelle-Sensor-Vorhersagewerterfassungsschritt, der das Erfassen eines berechneten Werts der VOC-Zuflussmenge von dem virtuellen Sensor, der für jedes RTO (100_n) in dem FID(110)-Verifizierungs- und Virtuelle-Sensor-Auswahlschritt ausgewählt wird, umfasst;

einen Sensordatenvergleichsschritt, der das wechselseitige Vergleichen des berechneten Werts der VOC-Zuflussmenge, die für jedes RTO (100_n) durch den Virtuelle-Sensor-Vorhersagewerterfassungsschritt erfasst wird, und des gesammelten Messwerts von dem FID (110) zum gleichen Zeitpunkt umfasst; und

einen Diagnosealarmerzeugungsschritt, der das Erzeugen eines Diagnosealarms für den Messwert von dem FID (110) umfasst, wenn es eine Differenz zwischen dem berechneten Wert der VOC-Zuflussmenge und dem Messwert von dem FID (110) um einen vorbestimmten Referenzwert oder mehr als ein Ergebnis des Vergleichs gibt.

7. Verfahren nach Anspruch 6, bei dem in dem FID(110)-Verifizierungs- und Virtuelle-Sensor-Auswahlschritt die erste virtuelle Sensoreinheit (210) ausgewählt wird, wenn der FID (110) vorhanden ist und die Zuverlässigkeit des FID (110) größer oder gleich einer vorbestimmten Referenz ist, und die zweite virtuelle Sensoreinheit (220) ausgewählt wird, wenn der FID (110) nicht vorhanden ist oder die Zuverlässigkeit des FID (110) kleiner als der vorbestimmte Referenzwert ist.

8. Verfahren nach Anspruch 7, bei dem die erste virtuelle Sensoreinheit (210) konfiguriert ist, um die VOC-Zuflussmenge unter Verwendung von RTO-Betriebsdaten durch ein auf künstlicher Intelligenz basierendes neuronales Netzwerkmodell zu berechnen, das unter Verwendung der Messwerte von dem FID (110) und der RTO-Betriebsdaten, die den Messwerten von dem FID (110) entsprechen, trainiert ist.

9. Verfahren nach Anspruch 7, bei dem die zweite virtuelle Sensoreinheit (220) die VOC-Zuflussmenge basierend auf der Differenz zwischen der Verbrennungsenergie des Kraftstoffs und dem Lufttemperaturanstieg in dem RTO (100_n) vorhersagt.

**Revendications**

1. Système de capteur virtuel destiné à mesurer une quantité de flux d'entrée de composés organiques volatils (COV) d'un oxydateur thermique régénératif (RTO), comprenant :

   une pluralité de RTO (oxydateur thermique régénératif) (100_n) et
   un dispositif de commande de RTO (200) pour commander les RTO (100_n),
   **caractérisé en ce que**
   chaque RTO (100_n) comprend une unité de mesure de données de fonctionnement (120) configurée pour mesurer les données de fonctionnement de RTO en temps réel à intervalles réguliers, et un détecteur à ionisation de flamme (FID) (110) pour mesurer régulièrement la quantité de flux entrant de COV en temps réel, dans lequel le dispositif de commande de RTO (200) comprend

   une unité de vérification de fiabilité (230) qui vérifie la présence ou l'absence ou la fiabilité du FID (110),
   une première unité de capteur virtuel (210) équipée d'un premier modèle de calcul de COV pour estimer la quantité de flux entrant de COV à partir des données de fonctionnement de RTO, le premier modèle de calcul de COV étant un modèle d'apprentissage automatique formé par des données de fonctionnement du RTO (100_n) et la quantité de COV mesurée par le FID (110) étant la vraie valeur,
   une deuxième unité de capteur virtuel (220) équipée d'un deuxième modèle de calcul de COV pour prédire le flux entrant de COV sur la base de la différence entre l'énergie de combustion du carburant et l'élévation de la température de l'air au RTO(100_n),
   une unité de commande (240) qui peut sélectionner soit la première unité de capteur virtuel (210), soit la deuxième unité de capteur virtuel (220) en fonction du résultat de vérification de l'unité de vérification de fiabilité (230) et de la présence ou l'absence du FID (110) pour chaque RT0(100_n) et y calculer et acquérir la quantité de flux entrant de COV.

2. Système de capteur virtuel selon la revendication 1, dans lequel
   l'unité de vérification de fiabilité (230) détermine que
   la fiabilité du FID (110) est faible si l'une des conditions suivantes est satisfaite : un cas où une valeur de mesure de flux entrant d'entrée de COV à partir du FID (110) de chaque RT0(100_n) ne change pas pendant une durée prédéterminée ou un cas dans lequel la valeur de mesure de flux entrant de COV excède une valeur de référence même s'il n'y a pas de changement au-delà de la plage de référence des données de fonctionnement de RTO.

3. Système de capteur virtuel selon la revendication 1, dans lequel
   l'unité de commande (240) sélectionne la première unité de capteur virtuel (210) quand il est déterminé que le FID (110) existe et que la fiabilité du FID (110) est élevée, et sélectionne la deuxième unité de capteur virtuel (220) quand il est déterminé que le FID (110) n'existe pas ou que la fiabilité du FID (110) est faible.

4. Système de capteur virtuel selon la revendication 1, dans lequel
   l'unité de commande (240) comprend en outre
   une unité de génération d'alarme de diagnostic qui compare la quantité de flux entrant de COV acquise à partir de l'unité de capteur virtuel sélectionnée à la valeur de mesure de flux entrant de COV du FID (110) au même point dans le temps, et génère une alarme de diagnostic pour la valeur de mesure de flux entrant de COV sur la base du résultat de comparaison.

5. Système de capteur virtuel selon la revendication 1, dans lequel

le premier modèle de calcul de COV de la première unité de capteur virtuel (210) est un modèle de réseau neuronal à base d'intelligence artificielle.

6. Procédé de vérification d'un FID (110) d'un RTO (100_n) en utilisant un système de capteur virtuel selon la revendication 1, le procédé comprend :

une étape de collecte et de mesure de données, qui consiste à collecter des données de fonctionnement et une valeur de mesure à partir du FID (110) mesurée en temps réel à intervalles réguliers par chaque RTO d'une pluralité de RT0(100_n) ;

une étape de vérification du FID (110) et une étape de sélection de capteur virtuel qui consistent à vérifier la fiabilité du FID (110) du RTO(100_n) sur la base de la valeur des mesures collectées du FID (110) pour chaque RT0(100_n) et à sélectionner soit la première unité de capteur virtuel (210), soit la deuxième unité de capteur virtuel (220) sur la base du résultat de vérification ;

une étape d'acquisition de valeur de prédiction de capteur virtuel qui consiste à acquérir une valeur calculée de la quantité de flux entrant de COV à partir du capteur virtuel sélectionné pour chaque RTO (100_n) à l'étape de vérification du FID (110) et de sélection de capteur virtuel ;

une étape de comparaison de données de capteur qui consiste à comparer mutuellement la valeur de la quantité de flux entrant de COV calculée acquise pour chaque RT0(100_n) par l'étape d'acquisition de valeur de prédiction de capteur virtuel et la valeur de mesure collectée à partir du FID (110) au même point dans le temps ; et

une étape de génération d'alarme de diagnostic qui consiste à générer une alarme de diagnostic pour la valeur de mesure à partir du FID (110) quand il y a une différence entre la valeur calculée de la quantité de flux entrant de COV et la valeur de mesure à partir du FID (110) qui est une valeur de référence prédéterminée ou plus, étant un résultat de la comparaison.

7. Procédé selon la revendication 6, dans lequel, dans l'étape de vérification du FID (110) et de sélection de capteur virtuel, la première unité de capteur virtuel (210) est sélectionnée si le FID (110) existe et que la fiabilité du FID (110) est supérieure ou égale à une référence prédéterminée et la deuxième unité de capteur virtuel (220) est sélectionnée si le FID (110) n'existe pas ou que la fiabilité du FID (110) est inférieure à la valeur de référence prédéterminée.

8. Procédé selon la revendication 7, dans lequel la première unité de capteur virtuel (210) est configurée pour calculer la quantité de flux entrant de COV en utilisant des données de fonctionnement de RTO par un modèle de réseau neuronal d'intelligence artificielle formé en utilisant la valeur de mesures à partir du FID (110) et les données de fonctionnement de RTO correspondant aux valeurs de mesure à partir du FID (110).

9. Procédé selon la revendication 7, dans lequel la deuxième unité de capteur virtuel (220) prédit la quantité de flux entrant de COV sur la base de la différence entre l'énergie de combustion du carburant et l'élévation de la température de l'air à l'intérieur du RTO (100_n).

**Fig. 1**

$100\_1$ — RTO$_1$

$100\_2$ — RTO$_2$

$\vdots$

$100\_n$ — RTO$_n$

200

RTO CONTROL DEVICE

**Fig. 2**

(a)

(b)

Fig. 3

EP 4 283 293 B1

**Fig. 4**

(a)

(b)

Fig. 5

Fig. 6

**EP 4 283 293 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101607878 B1
- KR 20210019909 A